# EUROPEAN PATENT SPECIFICATION

(11) **EP 3 003 236 B1**
(45) Date of publication and mention of the grant of the patent: **15.05.2019**
(21) Application number: 14807740.7
(22) Date of filing: 04.06.2014
(51) Int. Cl.: A61F 13/02, A61L 15/22, A61M 39/16, A61M 5/32

(54) **PROPHYLACTIC DRESSING AND USE OF SAME IN THE PREVENTION OF INFECTION**
PROPHYLAKTISCHER WUNDVERBAND UND VERWENDUNG DAVON ZUR VORBEUGUNG VON INFEKTIONEN
PANSEMENT PROPHYLACTIQUE ET SON UTILISATION DANS LA PRÉVENTION D'UNE INFECTION

(30) Priority: 04.06.2013 US 201361831018 P; 03.06.2014 US 201414294945
(43) Date of publication of application: 13.04.2016
(73) Proprietor: Gui Global Products, Ltd., Houston, TX 77056 (US)
(72) Inventor: SIMMONS, Kathleen, A., Houston, TX 78049 (US); MAYFIELD, Walter, G., Houston, TX 77042 (US); VALDEZ, Daniel, M., Kingwood, TX 77345 (US)
(74) Representative: Vigars, Christopher Ian
(86) International application number: PCT/US2014/040878
(87) International publication number: WO 2014/197572

(56) References cited:
- EP-A2- 0 236 104
- WO-A1-94/15562
- WO-A1-02/100639
- US-A- 3 918 446
- US-A- 4 040 427
- US-A- 4 966 590
- US-A- 5 607 388
- US-A1- 2007 003 606
- US-A1- 2010 294 286
- US-B2- 7 759 537

## Description

### TECHNICAL FIELD

The present invention relates to a dressing. The present invention particularly relates to a dressing having prophylactic properties.

### BACKGROUND

According to the Center for Disease Control, central line associated bloodstream infections result in thousands of deaths each year and billions of dollars in added costs to the U. S. healthcare system. Any transcutaneous or percutaneous medical device can lead to such infections. It would be desirable in the art treating wounds and caring for patients having transcutaneous or percutaneous devices to have a prophylactic dressing capable of preventing infection caused by contamination of the wound or the point where the medical device passes through the skin.

### SUMMARY

Aspects of the present invention are set out in the attached claims.

In one particular aspect, the invention is a dressing comprising a liquid resistant sheet and disposed thereon first and second adhesive strips, wherein the first and second adhesive strips are concentric; are located at or near the periphery of the liquid resistant sheet; are separated from one another by a gap; and are impermeable to liquids. In an aspect of the present invention, the gap is filled with a liquid indicator.

### BRIEF DESCRIPTION OF THE DRAWINGS

FIG. 1 is an illustration of a first embodiment of a dressing of the disclosure.
FIG 2A illustrates a first part of an embodiment of a dressing configured to be used with a catheter or other temporary appliance.
FIG 2B illustrates the use of 2A with an IV.
FIG 3 is a side view of a lead foam support using a 2 part dressing.
FIG 4 is a side view of a lead foam support including a strap to hold the lead or leads in place.
FIG. 5 is an illustration of an embodiment similar to that in FIG. 1 additionally comprising a surface treated to be resistant to sticking to a wound.

It will be appreciated that the various Figures are not necessarily to scale and that certain features have been exaggerated for clarity and do not necessarily limit the features of the invention.

### DETAILED DESCRIPTION

For the purposes of this application, the term "transcutaneous" means existing across the depth of the skin but also may mean passing through and into deeper tissues. The term "percutaneous" means made, done, or effected through the skin, such as using a needle. While not generally used interchangeably in the medical arts, for the purposes of this application, these terms are effectively synonyms because the dressings of this application may be used with any type of wound to the skin (except as noted below) as well as any opening into the body no matter how the wound or opening may have occurred. Similarly, the use of the term transcutaneous modifying a device or appliance also means including a percutaneous device or appliance.

Central line associated bloodstream infections result in thousands of deaths each year and billions of dollars in added costs to the U. S. healthcare system. Any transcutaneous or percutaneous medical device can lead to such infections. In addition to central line catheters, these infections may be caused by abdominal drains such as those used in liver transplants and hernia repair. Temporary devices used to assist other patients such as the so called left ventricular assistant devices (LVAD)s are of particular concern because they also very expensive and can be damaged by infections.

To improve the quality of life of patients with transcutaneous or percutaneous devices or even "slow to heal wounds," it is important to protect against infection caused by liquid infiltration. Such infiltration often occurs in mundane situation such as bathing, showering or other forms of ablution. For example an aseptic or antiseptic field around a wound can be compromised when water from a bath or shower passes into a dressing carrying with it harmful bacteria.

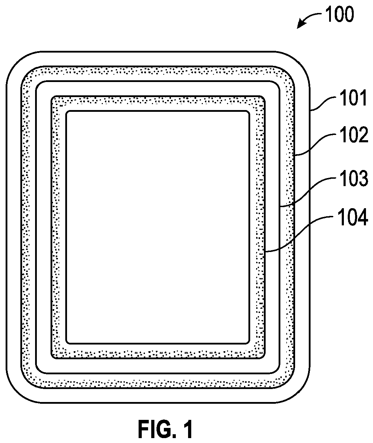

Employing a dressing of the disclosure can mitigate or prevent infiltration of environmental fluids into the wounds or insertion point of a transcutaneous or percutaneous device. Turning to **Figure 1**, a first embodiment of a dressing (**100**) of the disclosure is illustrated wherein (**101**) is a liquid resistant sheet. The liquid resistant sheet may be completely impermeable but in some embodiments may pass vapor while precluding the passage of liquids (sometime referred in the art as being able to breathe). Such sheets may be of one or more layers. In some embodiments, the liquid resistant sheet may be prepared from a polymeric material. Any material known to be useful in preparing such liquid resistant sheets to those of ordinary skill in the art may be used with the method of the disclosure.

Disposed upon the liquid resistant sheet is a first liquid barrier/adhesive strip (**102**). The first liquid barrier/adhesive strip is located closest to the edge of the liquid resistant sheet. The liquid barrier functions to prevent infiltration of liquid under the dressing. Exemplary adhesives useful with the dressings of the disclosure include but are not limited to Allevyn™ from Smith & Nephew, Largo, Fla., and Elasto-Gel[™ from Southwest Technologies, Inc., North Kansas City, Mo. Any adhesive known to those of ordinary skill in the art which are impermeable to liquids but also suitable for adhering a dressing to human skin may be employed in preparing the dressings of the disclosure.

Also shown in **Figure 1** is a second liquid barrier/adhesive strip (104). The second liquid barrier/adhesive strip is concentric with the first and they are separated from one another by a gap (**103**). The first and second liquid barrier/adhesive strip may be the same or different.

The gap between the first and second liquid barrier/adhesive strips is important. The gap is filled with a liquid indicator, and may also be filled with an absorbent, or an adsorbent material.

The use of an absorbent and/or a liquid indicator may permit for an extended period of prophylactic protection. When the gap is filled with an absorbent material, liquids that managed to infiltrate past the first liquid barrier/adhesive strip can be stopped by the absorbent materials for a period of time sufficient for the patient or the caregiver to perceive that the dressing has been compromised and should be replaced.

The use of a liquid indicator functions to make it easier to detect the infiltration of a liquid. The use of chromatic liquid indicators is especially useful as it allows for a very quick examination of the dressing. When the liquid indicator indicates that liquid has penetrated past the first liquid barrier/adhesive strip, the patient or a caregiver can make a ready determination that the dressing has been compromised and should be replaced.

The absorbent materials used with the method of the disclosure can be any known to be useful for preparing dressings. For example, in one embodiment a cellulose pad may be used. In another embodiment, a polymeric foam may be used for this purpose.

The liquid indicator may be any known to be useful to those of ordinary skill in the art of preparing dressings. It should be biologically benign and in some embodiments it may be hypoallergenic. While the indicator does not have to be a classical dye, if it is a dye and it is not proscribed for use on human skin, it may be used as long as it has a visible (chromatic) change in the presence of liquid water. In practicing the invention of the application, in some embodiments, the liquid indicator dye is a triarylmethane dye, a monoazo dye, a diazo dye, a xanthene dye, an anthraquinone dye, an indigoid dye, a quinoline dye, an FD&C dye, or a D&C dye. Such dyes useful with the present application include, but are not limited to: gentian violet, methylene blue, crystal violet, FD&C Yellow No. 5, FD&C Yellow No. 6, D&C Red NO. 17, FD&C Red No. 3, D&C Green No. 6, ethyl violet, brilliant green, FD&C Blue No. 2, D&C Yellow No. 1, FD&C Blue No. 1, or FD&C Green No. 3. IN some embodiments, it is desirable that the indicator turn blue on contact with water.

The dressings of the disclosure, especially when used with catheters and other transcutaneous or percutaneous devices may be composed of 2 parts. A first part that is applied either before the devices installed are also slipped underneath the lines leading from the device which functions to provide support to prevent rocking of the needle or catheter.

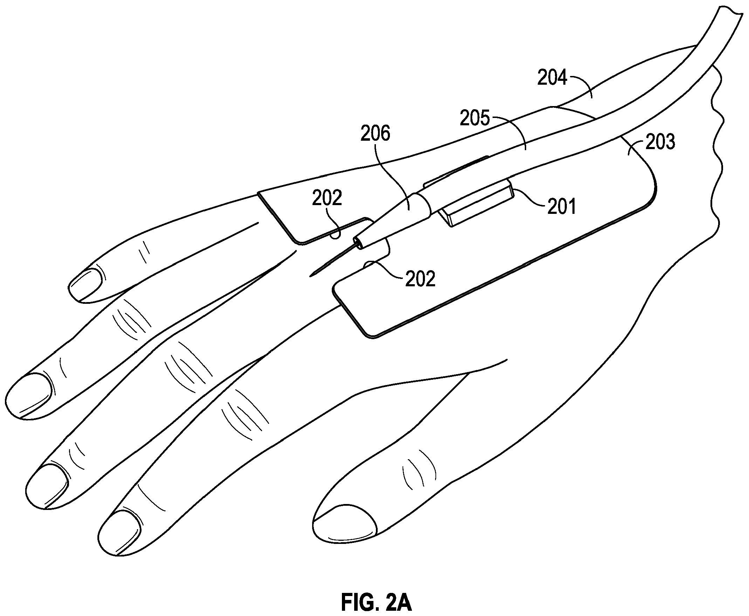

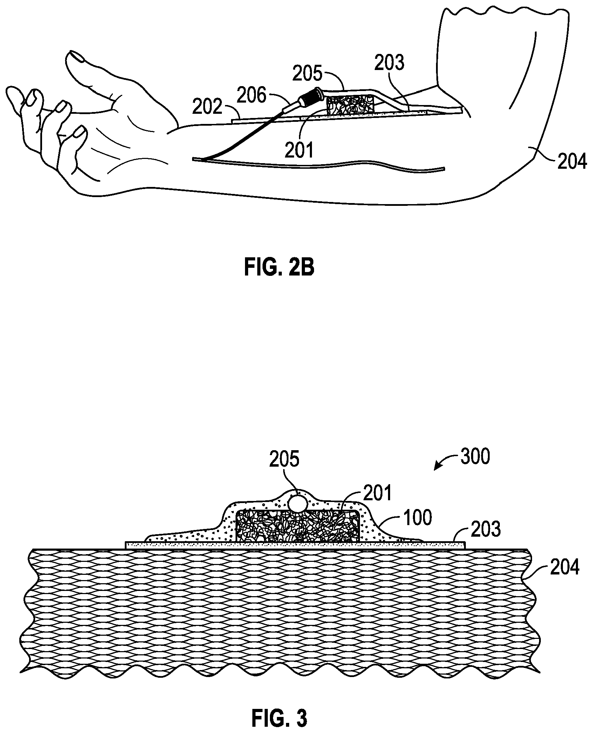

Turning to **Figures 2A** **&** **B**, the bottom piece of a two-part dressing is shown at (**200A**). In **Figure 2B**, the bottom part of the dressing is in place on a patient, held in place by an adhesive covering most or all of the bottom of the bottom dressing. A foam block (**201**) which functions to stabilize movement of the line (**205**) leading from the appliance (in this case an IV (**206**)) and also functions to create a seal with the top part of the dressing such as is illustrated in **Figure 1**. Note that there is a (**202**) that is sized to fit around the part of the appliance sitting on the surface of the patient (**204**). Beside stabilizing the needle are other part of the appliance, which reduces the amount of pain caused with movement of the line; the foam block allows the line to at least partially be submerged within the foam minimizing the amount of stretching required by the top of the dressing in order to make a liquid proof seal.

Turning to **Figure 3**, a two-part dressing of the disclosure is shown in place on a patient (**204**). The bottom part of the dressing (**200A**) including a foam pad (**201**) is illustrated wherein the foam pad both stabilizes the lead (**205**). The upper part of the dressing (**100**) is shown in place over the bottom part. It may be "baggy" or taut.

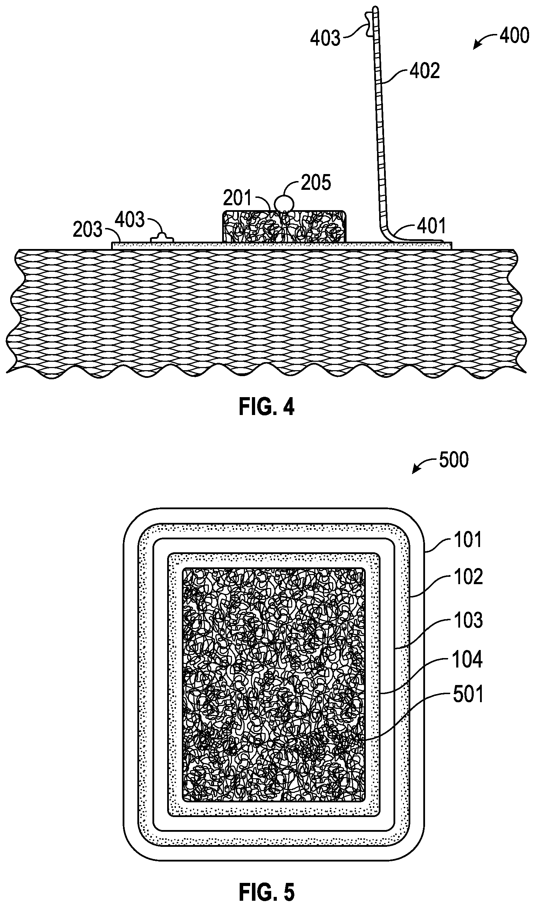

In an alternative embodiment, the bottom part of the two part dressing can be used even without the top portion. Turning to **Figure 4**, the bottom portion of the dressing illustrated in Figure 3 is shown with additional elements. This new element is a strap (**402**) affixed adjacent to or on the base of the foam pad with reference number (**401**) illustrating an attachment of the strap to the dressing surface. Reference number (**403**) shows a two part hook and eye attachment that functions to hold the strap in a folded over configuration when the two components are brought together. A tacky or other form of adhesive may be used in place of the hook and eye. When folded over a lead or leads, the strap secures the leads in place on the foam pad and also prevents the leads from being lifted up causing the device attached to the leads to press down on a needle or the transcutaneous or percutaneous device.

Turning now to **Figure 5**, therein is illustrated an embodiment similar to that of Figure 1 (**500**), but additionally comprising treating at least part of the dressing that would be in contact with a wound (**501**) with a composition that would prevent or at least reduce sticking. For example the part of the dressing in contact with the wound could be coated with or have integrated within it silica gel, or some other component to provide those properties. Any component that is safe for contact with a wound and which mitigate or prevent sticking to a wound may be used with the embodiments of the application. The area of non stick may encompass the entire inside surface of the dressing or it may be a narrow strip merely wide enough to cover at least part of the wound on which it is being used.

For the purpose of this application, the term lead or leads means an electrical wire or tubing for conveying a fluid, such as the line leading from an IV.

In alternative embodiments, the dressings of the disclosure can have separate apertures allowing for the exit of leads. Employment of antimicrobial elements is also within the scope of the disclosure. In one embodiment, the antimicrobial elements are delivered using a nano particle delivery system.

The dressings of the application are directed to the prevention of external liquid contamination of wounds or insertion points for transcutaneous or percutaneous devices. They are not suitable for use with wounds requiring maintaining a moist environment for the wounds. Exemplary of same would be ulcers and burns.
The dressings of the application are meant for temporary use. For example in some embodiments, they may be discarded after 5 days use. In other embodiments, they may be discarded after 2 days use. In still other embodiments, they may be used only to protect a patient for a single ablution and then discarded. They are also meant to be sized suitable for their intended applications with the dressing having dimensions suitable for its intended use.

## Claims

1. A dressing (100) comprising a liquid resistant sheet (101) and disposed thereon first and second adhesive strips (102,104), wherein the first and second adhesive strips (102,104) are concentric; are located at or near the periphery of the liquid resistant sheet (101); are separated from one another by a gap (103), and are impermeable to liquids, **characterised in that** the gap (103) is filled with a liquid indicator.

2. A dressing (100) as claimed in claim 1, wherein the liquid resistant sheet (101) is completely impermeable.

3. A dressing (100) as claimed in claim 1 , wherein the liquid resistant sheet (101) is able to pass vapor while precluding the passage of liquids.

4. A dressing (100) as claimed in any one of the preceding claims, wherein the liquid resistant sheet (101) is of one or more layers.

5. A dressing (100) as claimed in any one of the preceding claims, wherein the liquid resistant sheet (101) is prepared using a polymer.

6. A dressing (100) as claimed in claim 1, wherein the first and second adhesive strips (102, 104) comprise the same adhesive.

7. A dressing (100) as claimed in any one of the preceding claims, wherein the gap (103) is filled with an absorbent.

8. A dressing (100) as claimed in any one of the preceding claims, wherein the first adhesive strip (102) is located concentrically outwardly of the second adhesive strip (104).

9. A dressing (100) as claimed in any one of the preceding claims, wherein the liquid indicator is indicative of liquid penetrating past the first adhesive strip (102) into the gap (103).

10. A dressing (100) as claimed in any one of the preceding claims, wherein the liquid indicator is a chromatic liquid indicator.

11. A dressing (100) as claimed in any one of the preceding claims, wherein the liquid indicator is a dye selected from the group consisting of: triarylmethane dye, a monoazo dye, a diazo dye, a xanthene dye, an anthraquinone dye, an indigoid dye, a quinoline dye, an FD&C dye, and a D&C dye.

12. A dressing (100) as claimed in any one of the preceding claims, further comprising a bottom piece (200A).

13. A dressing (100) as claimed in claim 12, wherein the bottom piece (200A) comprises an adhesive sheet and affixed upon the side opposite the adhesive, a foam pad (201;401) wherein the foam pad (201;401) is configured to support leads (205) from a transcutaneous or percutaneous device.

14. A dressing (100) as claimed in claim 13, wherein the dressing further comprises a strap (402) which, when folded over a lead (205) or leads (205) secures the leads (205) in place on the foam pad (401).

15. A dressing (500) as claimed in any one of the preceding claims, wherein at least a part (501) of the dressing that will be in contact with a wound is treated with a composition to mitigate or stop the wound from sticking to the dressing.

## Patentansprüche

1. Wundverband (100), der eine flüssigkeitsbeständige Folie (101) und darauf angeordnet einen ersten und einen zweiten Klebestreifen (102, 104) umfasst, wobei der erste und der zweite Klebestreifen (102, 104) konzentrisch sind; sich am oder in der Nähe des Umfangs der flüssigkeitsbeständigen Folie (101) befinden; durch einen Zwischenraum (103) voneinander getrennt sind und für Flüssigkeiten undurchlässig sind, **dadurch gekennzeichnet, dass** der Zwischenraum (103) mit einem Flüssigkeitsindikator gefüllt ist.

2. Verband (100) nach Anspruch 1, wobei die flüssigkeitsbeständige Folie (101) vollständig undurchlässig ist.

3. Verband (100) nach Anspruch 1, wobei die flüssigkeitsbeständige Folie (101) Dampf hindurchtreten lassen kann, während sie den Durchtritt von Flüssigkeiten verhindert.

4. Verband (100) nach einem der vorhergehenden Ansprüche, wobei es sich bei der flüssigkeitsbeständigen Folie (101) um eine oder mehrere Schichten handelt.

5. Verband (100) nach einem der vorhergehenden Ansprüche, wobei die flüssigkeitsbeständige Folie (101) unter Verwendung eines Polymers hergestellt wird.

6. Verband (100) nach Anspruch 1, wobei der erste und der zweite Klebestreifen (102, 104) denselben Klebstoff umfassen.

7. Verband (100) nach einem der vorhergehenden Ansprüche, wobei der Zwischenraum (103) mit einem Absorptionsmittel gefüllt ist.

8. Verband (100) nach einem der vorhergehenden Ansprüche, wobei der erste Klebestreifen (102) sich konzentrisch nach außen von dem zweiten Klebestreifen (104) befindet.

9. Verband (100) nach einem der vorhergehenden Ansprüche, wobei der Flüssigkeitsindikator eine Flüssigkeit anzeigt, die an dem ersten Klebestreifen (102) vorbei in den Zwischenraum (103) eindringt.

10. Verband (100) nach einem der vorhergehenden Ansprüche, wobei der Flüssigkeitsindikator ein chromatischer Flüssigkeitsindikator ist.

11. Verband (100) nach einem der vorhergehenden Ansprüche, wobei der Flüssigkeitsindikator ein Farbstoff ist, der aus der Gruppe bestehend aus folgenden ausgewählt ist: Triarylmethanfarbstoff, ein Monoazofarbstoff, ein Diazofarbstoff, ein Xanthenfarbstoff, ein Anthrachinonfarbstoff, ein Indigoidfarbstoff, ein Chinolinfarbstoff, ein FD&C-Farbstoff und ein D&C-Farbstoff.

12. Verband (100) nach einem der vorhergehenden Ansprüche, der weiterhin ein Unterteil (200A) umfasst.

13. Verband (100) nach Anspruch 12, wobei das Unterteil (200A) eine Klebefolie und auf der Seite, die dem Klebstoff gegenüberliegt, angebracht ein Schaumstoffkissen (201; 401) umfasst, wobei das Schaumstoffkissen (201; 401) dazu konfiguriert ist, Leitungen (205) von einer transkutanen oder perkutanen Vorrichtung zu tragen.

14. Verband (100) nach Anspruch 13, wobei der Verband weiterhin einen Gurt (402) umfasst, der, wenn er über eine Leitung (205) oder mehrere Leitungen (205) gefaltet wird, die Leitungen (205) an Ort und Stelle auf dem Schaumstoffkissen (401) sichert.

15. Verband (500) nach einem der vorhergehenden Ansprüche, wobei mindestens ein Teil (501) des Verbands, der mit einer Wunde in Kontakt sein wird, mit einer Zusammensetzung behandelt ist, um ein Kleben der Wunde an dem Verband abzuschwächen oder zu unterbinden.

## Revendications

1. Pansement (100) comprenant une feuille résistante aux liquides (101) et disposées dessus une première et une seconde bandes adhésives (102, 104), où les première et seconde bandes adhésives (102, 104) sont concentriques ; sont situées à ou près de la périphérie de la feuille résistante aux liquides (101) ; sont séparées l'une de l'autre par un espace libre (103), et sont imperméables aux liquides, **caractérisé en ce que** l'espace libre (103) est rempli avec un indicateur de liquide.

2. Pansement (100) selon la revendication 1, dans lequel la feuille résistante aux liquides (101) est complètement imperméable.

3. Pansement (100) selon la revendication 1, dans lequel la feuille résistante aux liquides (101) est capable de faire passer de la vapeur tout en évitant le passage de liquides.

4. Pansement (100) selon l'une quelconque des revendications précédentes, dans lequel la feuille résistante aux liquides (101) est constituée d'une ou de plusieurs couches.

5. Pansement (100) selon l'une quelconque des revendications précédentes, dans lequel la feuille résistante aux liquides (101) est préparée en utilisant un polymère.

6. Pansement (100) selon la revendication 1, dans lequel les première et seconde bandes adhésives (102, 104) comprennent le même adhésif.

7. Pansement (100) selon l'une quelconque des revendications précédentes, dans lequel l'espace libre (103) est rempli avec un absorbant.

8. Pansement (100) selon l'une quelconque des revendications précédentes, dans lequel la première bande adhésive (102) est située concentriquement vers l'extérieur de la seconde bande adhésive (104).

9. Pansement (100) selon l'une quelconque des revendications précédentes, dans lequel l'indicateur de liquide est indicatif du liquide pénétrant après la première bande adhésive (102) dans l'espace libre (103).

10. Pansement (100) selon l'une quelconque des revendications précédentes, dans lequel l'indicateur de liquide est un indicateur de liquide chromatique.

11. Pansement (100) selon l'une quelconque des revendications précédentes, dans lequel l'indicateur de liquide est un colorant choisi dans le groupe constitué par : un colorant triarylméthane, un colorant monoazo, un colorant diazo, un colorant xanthène, un colorant anthraquinone, un colorant indigoïde, un colorant quinoléine, un colorant FD&C et un colorant D&C.

12. Pansement (100) selon l'une quelconque des revendications précédentes, comprenant en outre une pièce de fond (200A).

13. Pansement (100) selon la revendication 12,
dans lequel la pièce de fond (200A) comprend une feuille adhésive et fixé sur le côté opposé de l'adhésif, un coussinet de mousse (201;401) où le coussinet de mousse (201;401) est configuré pour supporter les conduites (205) d'un dispositif transcutané ou percutané.

14. Pansement (100) selon la revendication 13,
dans lequel le pansement comprend en outre une courroie (402) qui, quand elle est repliée sur une conduite (205) ou des conduites (205) attache les conduites (205) en place sur le coussinet de mousse (401).

15. Pansement (500) selon l'une quelconque des revendications précédentes, dans lequel au moins une partie (501) du pansement qui sera en contact avec une blessure est traitée avec une composition pour mitiger ou arrêter la blessure de coller au pansement.
